(19) Europäisches Patentamt
European Patent Office
Office européen des brevets

(11) Numéro de publication : **0 490 765 A2**

(12) # DEMANDE DE BREVET EUROPEEN

(21) Numéro de dépôt : **91403362.6**

(22) Date de dépôt : **12.12.91**

(51) Int. Cl.[5] : **C07D 207/273,** C07D 207/22, C07D 401/12, A61K 31/40, C07C 311/37, // C07C311:19

(30) Priorité : **13.12.90 IT 2237190**

(43) Date de publication de la demande : **17.06.92 Bulletin 92/25**

(84) Etats contractants désignés : **CH DE FR GB IT LI NL**

(71) Demandeur : **ROUSSEL-UCLAF**
**35, Boulevard des Invalides**
**F-75007 Paris (FR)**

(72) Inventeur : **Toja, Emilio**
**Via Plezzo 80**
**Milano (IT)**
Inventeur : **Bonetti, Carla**
**Via Beccalino**
**Fontanella(Bergamo) (IT)**
Inventeur : **Barzaghi, Fernando**
**Via Monterverdi 21**
**I-20052 Monza, Milan (IT)**
Inventeur : **Galliani, Giulio**
**13, Via Silva**
**1 Monza (IT)**

(74) Mandataire : **Tonnellier, Marie-José et al**
**111, route de Noisy B.P. no 9**
**F-93230 Romainville (FR)**

(54) **Dérivés de la 1-arylsulfonyl 2-pyrrolidone, leur procédé de préparation et les nouveaux intermédiaires obtenus, leurapplication comme médicaments et les compositions pharmaceutiques les renfermant.**

(57) L'invention concerne des composés :

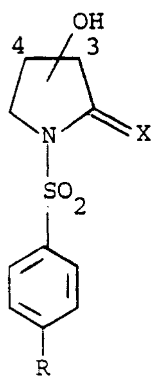

(I)

dans lesquels X représente O ou S, R représente

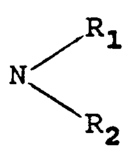

$R_1$ et $R_2$ représentant alkyle (→ 8C) ou formant avec N un hétérocycle renfermant éventuellement un autre hétéroatome, OH est en position 3 ou 4 ainsi que les dérivés fonctionnels de l'OH qui, in vivo, sont métabolisés en produits hydroxylés correspondants, leur préparation, leur application comme médicaments utiles pour leur activité anti-muscarinique.

EP 0 490 765 A2

La présente invention concerne de nouveaux dérivés de la 1-arylsulfonyl 2-pyrrolidone, leur procédé de préparation et les nouveaux intermédiaires ainsi obtenus, leur application comme médicaments et les compositions pharmaceutiques les renfermant.

L'invention a pour objet les composés de formule générale (I) :

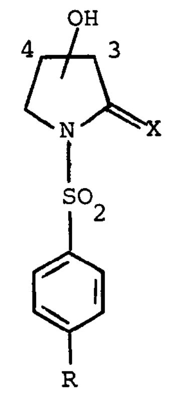

(I)

dans lesquels,

– le radical OH est en position 3 ou 4,

– X représente un atome d'oxygène ou de soufre,

– R en position para représente un radical

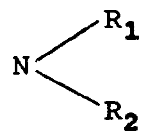

$R_1$ et $R_2$ identiques ou différents l'un de l'autre représentent un radical alkyle saturé ou insaturé, renfermant jusqu'à 8 atomes de carbone ou forment ensemble avec l'atome d'azote un radical hétérocyclique renfermant éventuellement un autre hétéroatome, ainsi que les dérivés fonctionnels de l'hydroxyle en 3 ou en 4 qui sont métabolisés in vivo en dérivés hydroxylés correspondants.

Les dérivés fonctionnels capables de générer OH "in vivo" peuvent être des esters dérivés d'acides organiques à chaînes linéaires ou ramifiées ou à chaînes aromatiques ou encore des esters dérivés d'acide inorganique comme l'acide phosphorique ou l'acide sulfurique.

Par radical alkyle, on entend de préférence un radical alkyle renfermant de 1 à 8 atomes de carbone, par exemple le radical méthyle, éthyle, propyle, isopropyle, butyle, isobutyle, tert-butyle, pentyle, hexyle, cyclopropyle, cyclobutyle, cyclopentyle ou cyclohexyle.

Par radical alkyle insaturé, on entend de préférence un radical éthényle, propényle et butényle.

Lorsque $R_1$ et $R_2$ forment avec l'atome d'azote auquel ils sont liés un radical hétérocyclique renfermant éventuellement un autre hétéroatome, il s'agit de préférence d'un radical pipéridyle, pipérazinyle, hexahydro azépinyle, morpholinyle ou pyrrolidinyle.

Parmi les composés préférés de l'invention, on peut citer :

– les composés dans lesquels le radical hydroxyle est sous forme libre et notamment ceux dans lesquels le radical hydroxyle est en position 4,

– les composés dans lesquels $R_1$ et $R_2$ forment ensemble avec l'atome d'azote auquel ils sont liés un radical hétérocyclique et notamment ceux dans lesquels le radical hétérocyclique est choisi dans le groupe constitué par les radicaux 1-pipéridinyle et hexahydro 1H-azépin-1-yle,

– les composés dans lesquels X représente un atome de soufre.

L'invention a naturellement plus particulièrement pour objet les composés dont la préparation est donnée ci-après dans la partie expérimentale et tout spécialement les composés des exemples 5, 6 et 7.

Les composés de formule (I) présentent d'intéressantes propriétés pharmacologiques et notamment une activité anti-muscarinique spécifique et sélective.

L'invention a donc pour objet les produits de formule (I) en tant que médicaments, utiles notamment pour le traitement des troubles spasmodiques divers en gastro-entérologie, en gynécologie, en obstétrique, en urologie, en hépatologie et en radiologie.

L'invention a plus particulièrement pour objet en tant que médicament, les composés préférés cités ci-dessus, à savoir les produits des exemples 5, 6 et 7.

La posologie usuelle est variable selon l'affection en cause, le sujet traité et la voie d'administration ; elle peut être comprise entre 10 mg et 1 g par jour, par exemple entre 30 et 60 mg par jour en une ou plusieurs prises pour le produit de l'exemple 5 administré par voie orale.

La présente invention a également pour objet les compositions pharmaceutiques renfermant comme principe actif au moins un produit de formule (I). Les compositions pharmaceutiques de l'invention peuvent être solides ou liquides et se présenter sous les formes pharmaceutiques couramment utilisées en médecine humaine, comme par exemple, les comprimés simples ou dragéifiés, les gélules, les granulés, les suppositoires, les préparations injectables ; elles sont préparées selon les méthodes usuelles.

Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

L'invention a également pour objet un procédé de préparation caractérisé en ce que l'on soumet un composé de formule (II) :

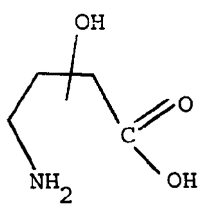

(II)

dans laquelle le radical OH est en position 2 ou 3 à l'action d'un composé de formule (III) :

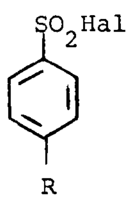

(III)

dans lequel Hal représente un atome d'halogène et R conserve sa signification précédente pour obtenir le composé de formule (IV) :

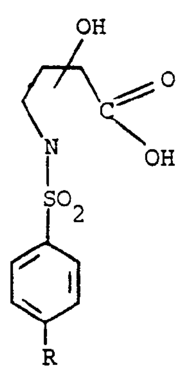

(IV)

que l'on soumet à l'action d'un agent de cyclisation pour obtenir un composé de formule ($I_A$) :

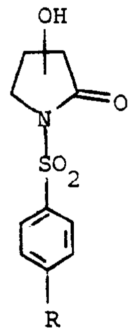

$(I_A)$

dans laquelle R a la signification précédente et OH est en position 3 ou 4, que l'on soumet, le cas échéant,

– soit à l'action d'un agent de fonctionnalisation du radical hydroxyle pour obtenir le composé de formule ($I_b$) correspondant :

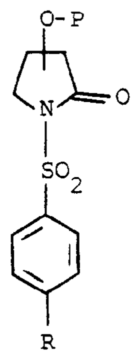

$(I_B)$

dans laquelle R a la signification précédente et P représente un groupement fonctionnel du radical hydroxyle,

– soit à l'action d'un agent de blocage de l'hydroxyle pour obtenir le composé de formule (V) :

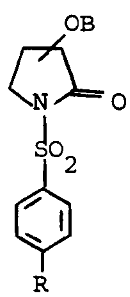

(V)

B représentant un groupement protecteur du radical hydroxyle, que l'on soumet à l'action d'un agent capable de convertir le radical carbonyle en radical thiocarbonyle pour obtenir le composé de formule (VI) :

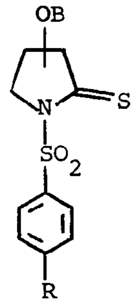

(VI)

que l'on soumet à l'action d'un agent de clivage du groupement protecteur du radical hydroxyle pour obtenir le composé de formule ($I_C$) correspondant :

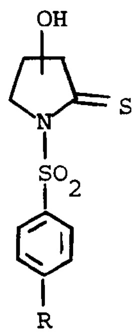

($I_C$)

dans laquelle R a la signification précédente et OH est en position 3 ou 4, que l'on soumet, le cas échéant, à l'action d'un agent de fonctionnalisation du groupement hydroxyle pour obtenir le composé de formule ($I_D$) correspondant.

Dans un mode de réalisation préférée du procédé de l'invention :

– la réaction du composé de formule (II) avec le composé de formule (III) est réalisée dans les conditions classiques de la réaction Schotten Baumann ou des variantes qui en découlent, en utilisant du diazabicyclooctane, une solution aqueuse d'hydroxyde alcalin, ou des bases organiques comme la pyridine,

– la cyclisation du composé de formule (IV) est réalisée en utilisant des agents de cyclisation choisis parmi : $(CF_3CO)_2O + CF_3CO_2Na$ ; $(CH_3CO)_2O + CH_3COONa$ ; $H_2SO_4+P_2O_5$ ; $PO_4H_3$ ; l'hexaméthyl disilazane en présence de chlorure de triméthylsilyle, le dicyclohexylcarbodiimide en présence de pyridine,

– le blocage de la fonction hydroxyle pour obtenir le produit de formule (V) est réalisé en protégeant la fonction hydroxyle par le groupement tert-butyldiméthylsilyle selon le procédé de Corey (J. Am. Chem. Soc. 1972, 94, 6190), ou tout autre dérivé du silyle comme il est décrit dans "Silicon in Organic Synthesis" Butterworths, London 1981, ou encore d'autres agents de blocage comme il est décrit dans Protective Groups in Organic Synthesis Wiley Interscience N.Y. 1981,

– l'agent capable de convertir le radical carbonyle en radical thiocarbonyle est notamment le réactif de Lawesson de formule

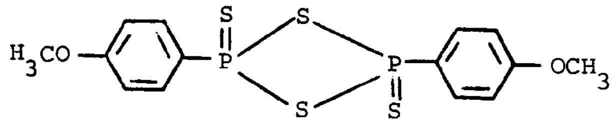

<(cf. TETRAHEDRON 41, 2567 (1985) ou TETRAHEDRON 41, 5061 (1985)>, au sein d'un solvant organique tel que le xylène, le toluène, le diméthoxyéthane, le tétrahydrofuranne ou le dioxane.

On peut également utiliser d'autres réactifs de thionation comme $P_2S_5$ ; $P_2S_5$-pyridine ; $P_2S_5$-TEA ; $P_2S_5$-$NaHCO_3$ ; $PCl_5$-$Alc_2S_3$-$Na_2SO_4$ ou encore le sulfure de bis(tricyclohexylétain) en présence de trichlorure de bore.

– le clivage du groupement protecteur de la fonction hydroxyle est effectué par exemple, au moyen du trifluorométhane sulfonate de triméthylsilyle au sein d'un solvant organique inerte selon le procédé de V. Bon et J. Villarasa (Tetrahedron Letters 1990, 31, 567-568) ou encore au moyen du fluorure de tétrabutylammonium au sein du tétrahydrofuranne,

– le dérivé fonctionnel ($I_B$) ou ($I_D$) peut être obtenu selon des conditions classiques connues de l'homme du métier. On peut utiliser par exemple comme agent d'estérification, un halogénure d'acide, de préférence un chlorure d'acide carboxylique tel que l'acide acétique, pivalique, hémisuccinique, benzoïque, alkyle ou dialkylamino acétique, nicotinique ou encore un dérivé d'acide phosphorique ou sulfurique.

L'invention a également pour objet une variante du procédé décrit précédemment, caractérisée en ce que l'on soumet un composé de formule (VII) :

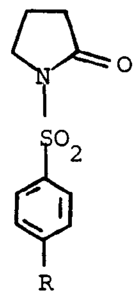

(VII)

dans laquelle R conserve sa signification précédente à l'action d'une base pour obtenir l'énolate de formule (VIII) :

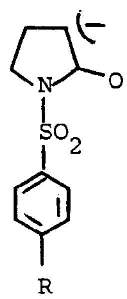

(VIII)

que l'on soumet à l'action d'un agent oxydant pour obtenir le composé de formule (I) correspondant dans lequel l'hydroxyle est libre, puis si désiré fonctionnalise ou si désiré transforme le radical carbonyle en radical thiocarbonyle comme indiqué précédemment, puis si désiré fonctionnalise le composé obtenu.

Dans un mode de réalisation préférée du procédé de l'invention :

– la base utilisée est choisie parmi le (bistriméthylsilyl) amidure de lithium de sodium ou de potassium, ou encore d'autres bases comme le diisopropylamidure de lithium, et l'on opère à basse température, au sein d'un solvant inerte comme le tétrahydrofuranne, l'éther, le dioxanne, le benzène, le toluène.

– l'agent d'oxydation utilisé est le 2-sulfonyl oxaziridine (cf Organic Synthesis Vol 66, 203-210 (1988). On peut également utiliser l'oxodiperoxymolybdenum (pyridine) hexamethyl phosphoryltriamide ou ou des peracides organiques comme l'acide métachloroperbenzoïque ou des peroxydes organiques comme le bis(trimethylsilyl) peroxyde ou le dibenzylperoxydicarbonate.

Les produits de formule (VII) utilisés comme produits de départ sont décrits et revendiqués dans le brevet européen n° 0335758.

Les produits obtenus lors de la mise en oeuvre du procédé de l'invention et de sa variante sont nouveaux et sont eux-mêmes un objet de la présente invention : il s'agit des produits de formule (IV), (V), (VI) et (VIII).

Les exemples suivants illustrent l'invention sans toutefois la limiter.

**<u>EXEMPLE 1</u> : 1-[[4-(diethylamino) phenyl] sulfonyl] 4-hydroxy 2-pyrrolidinone.**

<u>Stade A</u> : Acide 4-[[[4-(diéthylamino) phényl] sulfonyl] amino] 3-hydroxy butanoïque

A une solution de 1,19 g d'acide 3-hydroxy-4-amino butanoïque et 2,24 g de 1,4-diazabicyclo[2.2.2]octane

(DABCO) dans 10 cm³ d'eau, on ajoute une solution de 2,47 g de chlorure de 4-diéthyl aminobenzènesulfonyle dans 3,4 cm³ d'acétone : Réaction exothermique immédiate. On agite pendant 3 heures. On élimine l'acétone sous pression réduite puis ajoute à 0°C, 5 cm³ d'acide chlorhydrique 2 N. On extrait avec du chloroforme une fois 150 cm³ puis deux fois 100 cm³, lave à l'eau, sèche et évapore à sec sous pression réduite. On obtient 3,4 g de produit recherché.

Stade B :1-[[4-(diéthylamino) phényl] sulfonyl] 4-hydroxy-2-pyrrolidinone.

A une suspension de 11,6 g du produit obtenu comme au stade A dans 100 cm³ de benzène, on ajoute 50 cm³ d'anhydride trifluoroacétique et 4 g de trifluoroacétate de sodium et porte au reflux pendant 18 heures. On évapore à sec et reprend le résidu par du chlorure de méthylène , filtre l'insoluble et évapore à sec sous pression réduite. On dissout l'insoluble dans l'eau et extrait avec du chloroforme pour obtenir 1,8 g de produit cyclisé. Après évaporation du chloroforme, on obtient 17,19 g de sel trifluoroacétique du trifluoroacétate du produit attendu. On neutralise avec 20 cm³ d'une solution saturée de bicarbonate de sodium et on reprend avec 100 cm³ de méthanol, on agite 1 heure, ajoute de l'eau et filtre. Après cristallisation dans l'éthanol on obtient 5,23 g de produit brut et 2,5 g de produit supplémentaire à partir des eaux mères. Après chromatographie sur silice [éluant : acétate d'éthyle/hexane (8-2)], on obtient 6,25 g de produit recherché. F=165 °C recristallisé de l'acétate d'éthyle, et 1,48 g de produit comprenant une double liaison en position 3,4 de la pyrrolidinone.

| | | | | |
|---|---|---|---|---|
| **Analyse pour** :$C_{14}H_{20}N_2O_4S$. P.M : 312,39 | | | | |
| Calculés : | C% 53,83 | H% 6,45% | N% 8,97 | S% 10,26 |
| Trouvés : | 53,57 | 6,53 | 8,96 | |

**Exemple 2 : 1-[[4-(diméthylamino) phényl] sulfonyl] 4-(2,2-diméthyl 1-oxo propoxy) 2-pyrrolidinone.**

A une solution de 1 g de [1-(4-diéthylaminobenzènesulfonyl)-4-hydroxy-pyrrolidin-2-one] dans 20 cm³ de chlorure de méthylène on ajoute à 3°C 0,41 g de 1,4-diazabicyclo [2.2.2] octane (DABCO) et on ajoute lentement 0,43 g de chlorure de pivaloyle en solution dans 3 cm³ de chlorure de méthylène et laisse 16 heures à température ambiante. On ajoute un autre équivalent de chlorure de pivaloyle et de DABCO, laisse à température ambiante pendant 4 heures, amène à sec, reprend le résidu à l'acétate d'éthyle, lave à l'eau (à pH#6), filtre, amène à sec sous pression réduite et chromatographie le résidu sur silice (éluant : acétate d'éthyle/hexane 4-1), on obtient 0,6 g de produit attendu brut et 0,3 g de produit comprenant une double liaison en position 3,4 de la pyrrolidinone. Le produit brut est joint à une précédente venue pour obtenir après recristallisation dans un mélange acétate d'éthylehexane (3-50) 0,86 g du produit recherché. F = 117-118 °C.

**Exemple 3 : 1-[[4-(diéthylamino) phényl] sulfonyl] 4-hydroxy 2 pyrrolidine thione.**

Stade A : 1-[[4-(diéthylamino) phényl] sulfonyl] 4-[(1,1-diméthyléthyl) diméthyl tert-butylsilyloxy] 2-pyrrolidinone.

A un mélange de 1 g de [1-(4-diéthylamino benzène sulfonyle) 4-hydroxy pyrrolidin-2-one], 550 mg d'imidazole et 3 cm³ de diméthylformamide, on ajoute en 4 ou 5 fois : 0,6 g de diméthyltert-butyl chlorosilane. Après 3 heures à température ambiante, on élimine le solvant sous pression réduite, reprend avec de l'eau, extrait avec 3 fois 20 cm³ de chlorure de méthylène, sèche et évapore à sec sous pression réduite. On chromatographie le résidu sur silice (éluant : acétate d'éthyle-hexane 1-1), on obtient 1,31 g de produit recherché. F = 90-92 °C.

| | | | | | |
|---|---|---|---|---|---|
| **Analyse pour** : $C_{20}H_{34}N_2O_4S$ Si P.M. = 426,65 | | | | | |
| Calculés : | C% 56,30 | H% 8,03 | N% 6,57 | S% 7,52% | Si 6,58 |
| Trouvés : | 56,12 | 7,99 | 6,47% | | |

**Stade B : 1-[[4-(diéthylamino) phényl] sulfonyl] 4-[(1,1-diméthyléthyl) diméthylsilyloxy] 2-pyrrolidine thione**

On porte au reflux pendant quatre heures un mélange de 3,14 g du produit obtenu au stade A ci-dessus et 1,55 g de réactif de Lawesson dans 40 cm³ de toluène. On évapore le solvant sous pression réduite et chromatographie le résidu sur silice (éluant : acétate d'éthyle-hexane 3/7), on obtient 2,45 g de produit recherché. F = 136°C.(Après dissolution dans l'acétate d'éthyle, filtration, évaporation à sec, empâtage dans l'hexane.)

**Analyse pour** : $C_{20}H_{34}N_2O_3S_2$ Si P.M. = 442,72

| | | | | | |
|---|---|---|---|---|---|
| Calculés : | C% 54,26 | H% 7,74 | N 6,33 | S% 14,49 | Si 6,34 |
| Trouvés : | 54,13 | 7,73 | 6,28 | | |

Stade C : 1-[[4-(diéthylamino) phényl] sulfonyl] 4-hydroxy 2-pyrrolidine thione

A une solution de 1,3 g de 1-[[4-(diéthylamino) phényl] sulfonyl] 4-[(1,1-diméthyléthyl) diméthylsilyloxy] 2-pyrrolidine thione obtenu ci-dessus, dans 30 cm³ de chlorure de méthylène, on ajoute à -20°C en une heure 30 cm³ de bicarbonate de sodium, agite seize heures à température ambiante, décante, extrait avec du chlorure de méthylène, sèche et évapore à sec. Après cristallisation dans un mélange chloroforme-hexane on obtient 700 mg de produit recherché.
F = 149-150°C.

**Analyse pour** : $C_{14}H_{20}N_2O_3S_2$ P.M. = 328,46

| | | | | |
|---|---|---|---|---|
| Calculés : | C% 51,20 | H% 6,14 | N% 8,53 | S% 19,52 |
| Trouvés : | 50,66 | 6,08 | 8,39 | |

**Exemple 4** : 4-hydroxy 1-[[4-(1-pipéridinyl) phényl] sulfonyl] 2-pyrrolidinone

Stade A : Acide [3-hydroxy 4-[[4-(1-pipéridinyl) phényl] sulfonyl] amino] butanoïque.

A une solution de 1,07 g d'acide 3-hydroxy-4-amino butanoïque et 2,08 g de 1,4-diazabicyclo[2.2.2]octane (DABCO) dans 10 cm³ d'eau, on ajoute 2,34 g de chlorure de [4-(pipéridinyl) benzènesulfonyle] et 15 cm³ d'acétone. On agite 6 heures à température ambiante et laisse au repos 16 heures, on élimine l'acétone, refroidit à 0°C et ajoute 4,5 cm³ d'acide chlorhydrique 2N. On extrait avec 5 fois 60 cm³ de chloroforme, sèche et évapore à sec sous pression réduite. On obtient 3 g du produit attendu utilisé tel quel pour le stade suivant.

Stade B : 4-hydroxy 1-[[4-(1-pipéridinyl) phényl] sulfonyl] 2-pyrrolidinone

On porte au reflux, pendant 3 heures, 3 g du produit obtenu au stade A ci-dessus, 15 cm³ d'anhydride trifluoroacétique et 1,5 g de trifluoroacétate de sodium dans 50 cm³ de benzène. On évapore à sec et reprend par ajouts successifs de 60 cm³ de méthanol et 55 cm³ d'une solution à 5 % de bicarbonate de sodium dans le méthanol. On agite pendant 3 heures à température ambiante, évapore le méthanol à 30°C et concentre jusqu'à un volume de 30 cm³ . On extrait avec du chlorure de méthylène, sèche et évapore à sec sous pression réduite. On recueille 1,7 g de produit que l' on chromatographie sur silice (éluant : acétate d'éthyle-hexane (4-1). On obtient 0,95 g de produit attendu précipité de l'acétate d'éthyle avec de l'hexane. F = 158-159°C.

**Analyse pour** : $C_{15}H_{20}N_2O_4S$ P.M. = 324,40

| | | | | | |
|---|---|---|---|---|---|
| Calculés : | C% 55,54 | H% 6,21 | N% 8,64 | O% 19,73 | S% 9,88 |
| Trouvés : | 55,36 | 6,17 | 8,61 | | |

**Exemple 5 : 4-hydroxy-1-[[(4-(1-pipéridinyl) phényl] sulfonyl]-2-pyrrolidine thione**

Stade A : 4-[[(1,1-diméthyléthyl) diméthylsilyoxy] 1-[[4-(1-pipéridinyl) phényl] sulfonyl] 2-pyrrolidinone

A une solution de [1-(4-pipéridin-1-yl) benzènesulfonyl-4-hydroxy pyrrolidine 2 one] et 1,37 g d'imidazole dans 10 cm³ de diméthylformamide, on ajoute, par portions, 1,50 g de tert-butyl diméthyl chlorosilane et on agite 3 heures à température ambiante. On évapore le solvant sous pression réduite, reprend avec un peu d'eau et extrait avec de l'acétate d'éthyle, sèche et évapore à sec sous pression réduite. On chromatographie le résidu sur silice (éluant : acétate d'éthyle-n-hexane (2-3), on obtient 2,88 g du produit recherché. F = 111-112°C.

**Analyse pour** : $C_{21}H_{34}N_2O_4S$ Si P.M. = 438,67

| | | | | | |
|---|---|---|---|---|---|
| Calculés : | C% 57,50 | H% 7,81 | N% 6,39 | S% 7,31 | Si% 6,40 |
| Trouvés : | 57,66 | 7,85 | 6,54% | | |

Stade B : 4-[[(1,1-diméthyléthyl) diméthylsilyloxy] 1-[[4-(1-pipéridinyl) phényl] sulfonyl] 2-pyrrolidine thione

On porte au reflux pendant 3 heures 15 un mélange de 2,7 g du produit obtenu au stade A ci-dessus avec 1,55 g de réactif de Lawesson dans 25 cm³ de toluène. On refroidit, élimine le solvant et chromatographie le résidu sur silice (éluant : acétate d'éthyle-n-hexane (3-7). On obtient 2,32 g du produit recherché. F = 152,5-153°C après dissolution dans l'acétate d'éthyle et précipitation avec de l'hexane.

**Analyse pour** : $C_{21}H_{34}N_2O_3S_2$ Si P.M. = 454,73

| | | | | | |
|---|---|---|---|---|---|
| Calculés : | C% 57,47 | H% 7,54 | N% 6,16 | S% 14,10 | Si% 6,18 |
| Trouvés : | 57,71 | 7,62 | 6,22 | | |

Stade C : 4-hydroxy-1-[[(4-(1-pipéridinyl) phényl] sulfonyl]-2-pyrrolidine thione

A une solution, de 2,2 g du produit obtenu au stade B ci-dessus dans 25 cm³ de chlorure de méthylène, refroidie à -20°C, on ajoute 2,44 cm³ de trifluorométhanesulfonate de triméthylsilyle, on maintient à - 20°C, pendant 1 heure trente minutes et ajoute à -10°C, 20 cm³ d'une solution à 5% de bicarbonate de sodium et une pointe de spatule de bicarbonate de sodium en branches afin d'obtenir un pH # de 8. On laisse revenir à température ambiante et après 2 heures extrait la phase aqueuse avec du chlorure de méthylène, sèche et amène à volume réduit, précipite avec de l'hexane et obtient 1,4 g de produit recherché. F = 168-169°C.

**Analyse pour** : $C_{15}H_{20}N_2O_3S_2$ P.M. = 340,47

| | | | | |
|---|---|---|---|---|
| Calculés : | C% 52,92 | H% 5,92 | N% 8,23 | S% 18,84 |
| Trouvés : | 52,61 | 5,83 | 8,16 | |

**Exemple 6 : 1-[[4-(hexahydro 1 H-azépin-1-yl) phényl] sulfonyl] 4-hydroxy-pyrrolidine**

Stade A : Acide 4-[[[4-(hexahydro 1 H-azépin-1-yl) phényl] sulfonyl] amino] 3-hydroxy butanoïque

A une solution de 1,4 g d'acide 3-hydroxy-4-amino butanoïque et 3,36 g de 1,4-diazabicyclo[2.2.2]octane (DABCO) dans 18 cm³ d'eau, on ajoute 4,1 g de chlorure de (4-hexahydro azépin-1-yl-benzènesulfonyl) puis 12 cm³ d'acétone. On agite 6 heures à température ambiante, évapore l'acétone, refroidit à 0°C et ajoute 7,5 cm³ d'acide chlorhydrique 2N. On extrait avec du chloroforme, sèche et évapore à sec. On obtient 6,3 g de produit recherché utilisé tel quel pour le stade suivant.

Stade B : 1-[[4-(hexahydro 1 H-azépin-1-yl) phényl] sulfonyl] 4-hydroxy-pyrrolidinone

On agite pendant 12 heures au reflux 6,3 g du produit obtenu au stade A ci-dessus, 30 cm³ d'anhydride trifluoroacétique et 6,3 g de trifluoroacétate sodique. On évapore à sec, refroidit et reprend aussitôt avec 120

cm³ d'une solution à 5% de bicarbonate de sodium puis avec 350 cm³ de méthanol. On agite pendant 3 heures à température ambiante, concentre à volume réduit à 30°C puis extrait avec du chlorure de méthylène, sèche et évapore. On chromatographie le résidu sur silice (éluant : acétate d'éthyle-hexane (8-2) après cristallisation dans l'éthanol, on obtient 2,1 g de produit recherché. F = 170-172°C et 0,3 g de dérivé delta-3,4 correspondant.

**Analyse pour** : $C_{16}H_{22}N_2O_4S$ P.M. = 338,43

| | | | | |
|---|---|---|---|---|
| Calculés : | C% 56,79 | H% 6,55 | N% 8,29 | S% 9,47 |
| Trouvés : | 56,55 | 6,59 | 8,38 | |

<u>Exemple 7</u> : **1-[[4-(hexahydro 1-H-azépin-1-yl) phényl] sulfonyl] 4-hydroxy pyrrolidine thione**

<u>Stade A</u> : 4-[[(1,1-diméthyléthyl) diméthylsilyloxy] 1-[[4-(hexahydro 1-H-azépin-1-yl) phényl] sulfonyl] 4-hydroxy-pyrrolidinone

A une solution de 2,5 g de [1-(4-(hexahydro 1-H-azépin-1-yl) benzènesulfonyl 4-hydroxy-pyrrolidin-2-one] et 1,27 g d'imidazole dans 10 cm³ de diméthylformamide, on ajoute par portions 1,38 g de tert-butyl diméthylchlorosilane et on agite pendant 1 heure 30. On évapore le solvant, reprend avec 10 cm³ d'eau et extrait avec du chlorure de méthylène, sèche et évapore à sec. Après chromatographie sur silice (éluant : acétate d'éthyle-hexane 4-6), on obtient 3,1 g du produit recherché. F = 121°C.

**Analyse pour** : $C_{22}H_{36}N_2O_4S$ Si P.M. = 452,69

| | | | | | |
|---|---|---|---|---|---|
| Calculés : | C% 58,37 | H% 8,02 | N% 6,19 | S% 7,08 | Si% 6,20 |
| Trouvés : | 58,58 | 7,91 | 6,28 | | |

<u>Stade B</u> : 4-[(1,1-diméthyléthyl) diméthylsilyloxy] 1-[[4-(hexahydro 1-H-azépin-1-yl) phényl] sulfonyl] 2-pyrrolidine thione

On porte au reflux pendant 4 heures 30 un mélange de 3 g du produit obtenu au stade précédent et 1,61 g de réactif de Lawesson dans 30 cm³ de toluène. On élimine le solvant et chromatographie le résidu sur silice (éluant : acétate d'éthyle-hexane 3-7), on obtient 2,5 g du produit attendu. F = 135-136°C (après empâtage dans l'hexane).

**Analyse pour** : $C_{22}H_{36}N_2O_3S_2Si$ P.M. = 452,69

| | | | | | |
|---|---|---|---|---|---|
| Calculés : | C% 56,37 | H% 7,74 | N% 5,98 | S% 13,68 | Si% 5,99 |
| Trouvés : | 56,63 | 7,69 | 5,91 | | |

<u>Stade C</u> : 1-[[4-(hexahydro 1-H-azépin-1-yl) phényl] sulfonyl] 4-hydroxy-pyrrolidine thione

A une solution de 2,3 g du produit obtenu au stade précédent dans 25 cm³ de chlorure de méthylène, on ajoute, à - 20°C, 2,6 cm³ de triflate de triméthylsilyle. On agite pendant 1 heure 30 à froid puis à -10°C, on ajoute 20 cm³ d'une solution à 5% de bicarbonate de sodium et un peu de bicarbonate de sodium en branches pour ajuster le pH à # 8. On agite 30 minutes à température ambiante, sépare la phase aqueuse et l'extrait avec du chlorure de méthylène, la sèche et évapore à sec sous pression réduite. On dissout le résidu dans le chloroforme, filtre et précipite par ajout de n-hexane, on obtient 1,5 g de produit recherché. F = 138-139°C.

**Analyse pour** : $C_{16}H_{22}O_3S_2$ P.M. = 354,49

| | | | | |
|---|---|---|---|---|
| Calculés : | C% 54,21 | H% 6,26 | N% 7,90 | S% 18,09 |
| Trouvés : | 54,02 | 6,21 | 7,86 | |

**Exemple 8 : 4-(benzyloxy) 1-[[4-diéthylamino) phényl] sulfonyl] 2-pyrrolidinone**

A une solution de 1,03 g de [1-(4-diéthylaminobenzène sulfonyle) 4-hydroxy pyrrolidin-2-one] dans 20 $cm^3$ de tétrahydrofuranne et à - 65°C, on ajoute 2,27 $cm^3$ d'une solution 1,6M de butyllithium dans l'hexane. On laisse 20 minutes à -70°C et ajoute lentement 0,51 g de chlorure de benzoyle dans 3 $cm^3$ de tétrahydrofuranne puis laisse revenir à température ambiante et agite 30 minutes. On évapore à sec, reprend le résidu dans l'eau, extrait à l'acétate d'éthyle, élimine le solvant et chromatographie le résidu sur silice (éluant : acétate d'éthyle-n-hexane 1-1), on obtient 1,1 g de produit brut contenant un peu de dérivé delta-3,4 que l'on cristallise dans l'acétate d'éthyle/hexane. On obtient 0,95 g de produit recherché. F = 118-120°C.

**Analyse pour** : $C_{21}H_{24}N_2O_5S$ P.M = 416,50

| | | | | |
|---|---|---|---|---|
| Calculés : | C% 60,56 | H% 5,81 | N% 6,73 | S% 7,70 |
| Trouvés : | 60,66 | 5,93 | 6,75 | |

**Exemple 9 : Di-(tert-butyle)phosphate de [1-[[4-diéthylamino) phényl] sulfonyl] 2-oxo pyrrolidin-4-yl.**

A une suspension de 1,03 g de [1-(4-diéthylaminobenzène sulfonyl) 4-hydroxy pyrrolidine-2-one] dans 10 $cm^3$ de tétrahydrofuranne, on ajoute 0,75 g de [di-tert-butyl-N,N-diéthyl phosphoramidate] (Synthesis (1988) 142-144) et en une fois 0,76 g de tétrazole, on agite 30 minutes à température ambiante et refroidit à -40°C. On ajoute rapidement 1,38 g d'acide métachloroperbenzoïque à 50-60% dans 7 $cm^3$ de chlorure de méthylène en maintenant la température inférieure à 0°C. On laisse revenir à température ambiante et agite 10 minutes ; on ajoute 10 $cm^3$ d'une solution de bisulfite de sodium à 10 % et après 10 minutes à température ambiante, ajoute 70 $cm^3$ d'ether diéthylique. On décante et lave la phase organique avec deux fois 20 $cm^3$ d'une solution de bisulfite de sodium à 10 % puis par deux fois avec du bicarbonate de sodium à 5 %, sèche et évapore à sec sous pression réduite. On chromatographie le résidu sur silice (éluant : chloroforme-acétone 4-1) et obtient 1 g de produit recherché.

**Analyse pour** : $C_{22}H_{37}N_2O_7PS$ P.M. = 504,59

Calculés : C% 52,37 H% 7,39 N% 5,55 P% 6,14 S% 6,35

**Exemple 10** : Phosphate de [1-[[4- (diéthylamino) phényl] sulfonyl] 2-oxo pyrrolidin-4-yl.

On agite pendant 4 heures le mélange de 1,51 g du produit obtenu à l'exemple 9 avec 30 $cm^3$ d'acide chlorhydrique N dans le dioxanne. On évapore à sec reprend avec un peu d' eau et extrait avec de l'acétate d'éthyle. On sèche puis évapore à sec sous pression réduite, on obtient 0,99 g de produit brut contenant un peu de dérivé delta-3,4 correspondant que l'on empâte dans l'acétate d' éthyle on recueille ainsi 0,80 g du produit recherché. F = 89-90°C.

**Analyse pour** : $C_{14}H_{21}N_2O_7PS$ P.M. = 392,37

| | | | | | |
|---|---|---|---|---|---|
| Calculés : | C% 42,86 | H% 5,39 | N 7,14 | P% 7,89 | S% 8,17 |
| Trouvés : | 42,91 | 5,48 | 7,27 | | |

**Exemple 11 : 1-[[4-(diéthylamino) phényl] sulfonyl] 3-hydroxy 2-pyrrolidinone.**

A une solution de 9,3 g de [1-(4-diéthylaminophénylsulfonyle) pyrrolidin-2-one] préparée comme dans la demande de brevet européen n° E.P.A. 0 335 758 dans 280 $cm^3$ de tétrahydrofuranne refroidie à -78°C, on ajoute 31,2 $cm^3$ d'une solution molaire de sel de lithium d'hexaméthyldisilylazane en maintenant la température inférieure à -70°C. On agite 1 heure à -78°C puis ajoute en une fois 12,3g de 2-sulfonyloxaziridine (Org. Synthesis 66 203-210 (1988)) on agite 2 heures à -78°C. On laisse revenir à -5°C puis refroidit à -40°C et ajoute 150 $cm^3$ d'une solution aqueuse saturée de chlorure d'ammonium, on laisse revenir à température ambiante et ajoute 130 $cm^3$ d'une solution saturée de chlorure de sodium, on lave la phase aqueuse avec du chloroforme, sèche et évapore à sec sous pression réduite. On chromatographie le résidu sur silice (éluant : acétate d'éthyle-hexane (1-1)) on obtient 2,6 g de produit que l'on recristallise deux fois dans l'isopropanol pour recueillir

1,8 g de produit recherché. F = 164-165°C.

**Analyse pour** : $C_{14}H_{20}N_2O_4S$ P.M. = 312,39

| | | | |
|---|---|---|---|
| Calculés : | C% 53,83 | H% 6,45 | N% 8,97 |
| Trouvés : | 53,91 | 6,50 | 8,94 |

**Exemple 12 : 1-[[4-(diéthylamino) phényl] sulfonyl] 3-hydroxy 2-pyrrolidin-2-thione.**

En opérant comme à l'exemple 3, à partir du composé obtenu à l'exemple ci-dessus, on a préparé le produit recherché.

En opérant comme à l'exemple 8, en utilisant les réactifs appropriés on a obtenu les composés des exemples suivants :

**Exemple 13 : 3-méthoxycarbonyl propanoate de 1-[[4-diéthylamino) phényl] sulfonyl] 2-pyrrolidinone-4-yl.**

**Analyse pour** : $C_{19}H_{26}N_2O_7S$ P.M. = 426,49

| | | | |
|---|---|---|---|
| Calculés : | C% 53,51 | H% 6,14 | N% 6,57 |
| Trouvés : | 53,68 | 6,12 | 6,42 |

**Exemple 14 : benzyle carbonate de 1-[[4-diéthylamino) phényl] sulfonyl] 2-pyrrolidinone-4-yl.**

F = 105-106°C.

**Analyse pour** : $C_{22}H_{26}N_2O_6S$ P.M. = 446,53

| | | | |
|---|---|---|---|
| Calculés : | C% 59,18 | H% 5,87 | N% 6,27 |
| Trouvés : | 59,33 | 5,78 | 6,16 |

**Exemple 15 : 4-[(3-pyridin)carbonyloxy]-1-[[4-diéthylamino) phényl]sulfonyl] 2-pyrrolidinone.**

F = 134-135°C.

**Analyse pour** : $C_{20}H_{23}N_3O_5S$ P.M. = 417,49

| | | | |
|---|---|---|---|
| Calculés : | C% 57,54 | H% 5,55 | N% 10,07 |
| Trouvés : | 57,75 | 5,67 | 10,18 |

**Exemple 16 : 4-acétoxy-1-[[4-diéthylamino) phényl] sulfonyl] 2-pyrrolidinone.**

F = 92-93°C.

**Analyse pour** : $C_{16}H_{22}N_2O_5S$ P.M. = 354,43

| | | | |
|---|---|---|---|
| Calculés : | C% 54,22 | H% 6,26 | N% 7,90 |
| Trouvés : | 54,45 | 6,37 | 8,03 |

**Exemples de compositions pharmaceutiques.**

a) On a préparé des comprimés répondant à la formule suivante :

- Produit de l'exemple 5 ........................ 10 mg
- Excipient q.s. pour un comprimé terminé à ...... 300 mg

(Détail de l'excipient : lactose, amidon de blé, amidon traité, amidon de riz, stéarate de magnésium, talc).

b) On a préparé des gélules répondant à la formule suivante :

- Produit de l'exemple 7 ........................ 20 mg
- Excipient q.s. pour une gélule terminée à ...... 300 mg

(Excipient : talc, stéarate de magnésium, aérosil).

## ETUDES BIOCHIMIQUES ET PHARMACOLOGIQUES

1) **Liaison à des différents récepteurs cérébraux.**

a) Récepteur muscarinique 1.

Sa préparation est faite à partir de Cortex prélevés sur des cerveaux de rats mâles pesant 150 à 200 g, (Iffa Crédo) broyé au Polytron dans un tampon Na/K 10 mM pH 7,4. Après incubation (aliquotes de 0,5 ml d'homogénat) pendant 60 minutes à 25°C en présence de 0,25 nM de $^3H$ pirenzépine soit seule, soit avec le produit à tester, soit avec un excès de pirenzépine à $10^{-5}$M (pour déterminer la radioactivité fixée non spécifique), les incubats sont refroidis et filtrés.

La filtration est effectuée sur filtres Whatman GF/C prélavés dans une solution de polyéthylène imine à 0,05%. Les filtres sont rincés par 3 x 5 ml de tampon phosphate Na/K 10 mM pH 7,4 puis on effectue les mesures par scintillation liquide.

b) Récepteur muscarinique 2.

La préparation est effectuée à partir de cerveaux de rats mâles pesant 150 à 200 g (Iffa Crédo).

Les cerveaux sont broyés (Téflon-verre) dans une solution de sucrose 0,32 M. L'homogénat est centrifugé 10 minutes à 1000 g (0 - 4°C).

Le surnageant obtenu est recueilli et centrifugé à 30 000 g pendant 15 minutes (0 - 4°C).

Le culot est remis en suspension dans un tampon Tris 50 mM pH 7,5 et le nouvel homogénat est centrifugé de nouveau à 30 000 g pendant 15 minutes (0 - 4°C).

Les culots, après élimination du surnageant, peuvent être utilisés aussitôt ou conservés jusqu'à 1 mois à -30°C.

Pour une expérience les culots sont d'abord décongelés, si nécessaire, à température ambiante et remis en suspension à l'aide d'un Dounce dans du tampon Tris 50 mM pH 7,5. Des aliquotes de 2 ml sont mis à incuber 60 minutes à 25°C en présence de 0,3 nM de $^3H$ quinuclidinyl benzylate soit seul, soit avec le produit à tester, soit avec de la benzatropine à $10^{-5}$M pour déterminer la radioactivité fixée non spécifique.

A la fin de l'incubation, les tubes d'incubats sont refroidis à 4°C et filtrés rapidement sur filtres Whatman GF/C. Les filtres sont rincés par 3 x 5 ml de tampon Tris 50 mM pH 7,5 puis on effectue les mesures par scintillation liquide (Henry I Yamamura, Solomon H. Snyder, Proc. Nat. Acad. Sc. (1974) 71, n° 5, 1725-1729).

Les résultats sont exprimés en $CI_{50}$ (concentration nécessaire pour inhiber de 50% la radioactivité spécifique fixée).

**TABLEAU 1**

| Composé de l'exemple | Affinité pour les récepteurs muscariniques $M_1$ et $M_2$ | |
|---|---|---|
| | [$^3$H]pirenzépine | [$^3$H]quinuclidinyl benzylate |
| 1 | 370 | > 5 000 |
| 2 | 720 | > 5 000 |
| 3 | 370 | > 5 000 |
| 4 | 560 | 10 000 |
| 5 | 170 | > 5 000 |
| 6 | 62 | 4 500 |
| 7 | 83 | 10 000 |

Les composés des exemples 1 à 6 montrent une remarquable et intéressante affinité pour le récepteur muscarinique, et principalement pour le récepteur de type $M_1$. Par contre les mêmes composés ont montré une affinité négligeable ($CI_{50}$ > 5000-10000) pour les autres récepteurs examinés parmi lesquels ont peut citer ceux de la dopamine, de l'histamine, de la sérotonine (5 $HT_1$ et 5 $HT_2$), des benzodiazépines, du GABA, des adrénorécepteurs (alpha1, alpha2, béta1, béta2) ou encore les récepteurs opiacés (mu, kappa).

2) **Intéraction et affinité avec différents récepteurs intestinaux.**

L'intéraction des composés avec différents récepteurs a été évaluée sur l'iléon isolé du cobaye selon la méthode suivante.

Des segments d'iléon de cobayes de 2,5 - 3 cm ont été lavés et immédiatement suspendus dans un bain contenant 10 ml d'une solution de Tyrode à 37°C et aérée avec un mélange d'oxygène (95%) et de gaz carbonique (5%). Après une période de stabilisation de 30 minutes au moins, on enregistre les contractions, en maintenant la préparation sous tension constante de 1 g, à l'aide d'un capteur relié à un polygraphe. On a évalué l'action agoniste en laissant le composé en contact avec le tissu isolé pendant une période nécessaire à exprimer la contraction maximale ; ensuite, on a lavé avec la solution de Tyrode. On n'a ajouté la dose suivante au bain qu'après que la préparation fût revenue sur sa ligne de base. Comme produit de référence, on a employé l'arécoline. On a évalué l'action antagoniste sur les contractions induites par l'acétylcholine (1 x $10^{-6}$M), l'histamine (1 x $10^{-5}$M) et le chlorure de baryum (2 x $10^{-4}$M). L'atropine, le diphénydramine et la papavérine ont été employés comme produits de référence. Le temps de contact avant d'ajouter l'agoniste a été d'une minute.

Pour chaque composé les courbes dose-réponse sont obtenues avec 4 à 6 concentrations différentes et 3 à 5 épreuves indépendantes. L'activité agoniste est exprimée par le $pD_2$ (logarithme négatif de la dose qui produit 50% de l'effet maximum). L'activité antagoniste est exprimée par la $CI_{50}$ (concentration inhibant 50% de la réponse maximale).

Les résultats obtenus avec les composés des exemples 1 à 6 sont reportés dans le tableau suivant :

**TABLEAU 2**

| Composé de l'exemple | Antagoniste à différents agents ($CI_{50}$:M) | | | Action agoniste $pD_2$ |
|---|---|---|---|---|
| | ACh | Histam. | $BaCl_2$ | |
| 1 | $8,6x10^{-7}$ | $> 10^{-5}$ | $> 10^{-5}$ | < 4 |
| 2 | $5,5x10^{-6}$ | $> 10^{-5}$ | $> 10^{-5}$ | < 4 |
| 3 | $3,0x10^{-6}$ | $> 10^{-5}$ | $> 10^{-5}$ | < 4 |
| 4 | $1,2x10^{-6}$ | $> 10^{-5}$ | $2,5x10^{-6}$ | < 4 |
| 5 | $1,4x10^{-6}$ | $> 10^{-5}$ | $> 10^{-5}$ | < 4 |
| 6 | $1,1x10^{-7}$ | $> 10^{-5}$ | $2,2x10^{-6}$ | < 4 |
| 7 | $5,1x10^{-7}$ | $> 10^{-5}$ | $3,6x10^{-6}$ | < 4 |
| Atropine | $9,5x10^{-9}$ | | | |
| Diphénydramine | | $8,3x10^{-7}$ | | |
| Papavérine | | | $4,5x10^{-5}$ | |
| Arécoline | | | | 6,68 |

Les études "in vitro" sur l'iléon isolé de cobaye ont mis en évidence que les composés de l'invention sont des agents antimuscariniques. Ils antagonisent les contractions induites par l'acétylcholine mais non celles induites par l'histamine.

3) **Action anticholinergique "in vivo".**

L'activité anticholinergique des composés a été déterminée en évaluant la capacité d'inhiber les effets cholinomimétiques induits par le carbachol. Le sulfate d'atropine a été employé comme produit de référence.

On utilise des souris mâles $CD_1$ pesant 25 à 30 g. Elles sont réparties en groupes de 6 animaux et traitées par voie intrapéritonéale à des doses scalaires des produits ou 0,25% de Méthocel pour les témoins. On utilise 12 animaux pour chaque dose. 30 minutes après l'administration des composés, on injecte aux souris par voie sous-cutanée 1 mg/kg de carbachol, dissous dans du sérum physiologique.

Chaque animal a été examiné 30 minutes après l'injection de carbachol pour évaluer la présence de diarrhée, salivation et larmoiement ; on a mesuré aussi la température corporelle au moyen d'un thermocouple inséré de 1,5 cm dans le rectum.

Le carbachol (1 mg/kg s.c.) a induit diarrhée, salivation et larmoiement chez toutes les souris témoins et une diminution de la température rectale de 2,5°C environ.

Pour chaque composé, nous avons déterminé la dose capable d'inhiber chez 50 % des animaux, l'apparition des symptômes cholinomimétiques induits par le carbachol et d'augmenter de 1°C l'effet hypothermique induit par l'agent cholinergique.

**TABLEAU 3**

| Composé de l'exemple | Dose mg/kg i.p. Diarrhée | salivation | larmoiement | Température corporelle |
|---|---|---|---|---|
| 5 | 6 | 50 | > 50 | 25 |
| 6 | 12 | > 50 | > 50 | > 50 |
| 7 | 5 | 50 | > 50 | 20 |
| Atropine | 0,04 | 0,06 | 0,05 | 0,03 |

Les résultats obtenus montrent que, contrairement à l'atropine, les composés de l'exemple 5, 6 et 7 exercent "in vivo" une action anticholinergique sélective au niveau de la musculature intestinale.

## Revendications

**1)** Les composés de formule générale (I) :

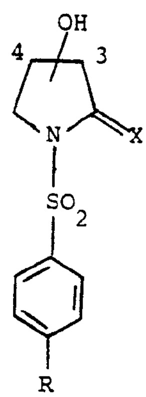

(I)

dans lesquels,

– le radical OH est en position 3 ou 4,
– X représente un atome d'oxygène ou de soufre,
– R en position para représente un radical

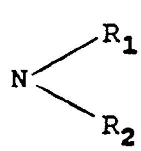

$R_1$ et $R_2$ identiques ou différents l'un de l'autre représentent un radical alkyle saturé ou insaturé, renfermant jusqu'à 8 atomes de carbone ou forment ensemble avec l'atome d'azote un radical hétérocyclique renfermant éventuellement un autre hétéroatome, ainsi que les dérivés fonctionnels de l'hydroxyle en 3 ou en 4 qui sont métabolisés in vivo en dérivés hydroxylés correspondants.

**2)** Les composés de formule (I) tels que définis à la revendication 1, dans lesquels le radical hydroxyle est sous forme libre.

**3)** Les composés de formule 1 tels que définis à la revendication 1 ou 2, dans lesquels le radical hydroxyle est en position 4.

**4)** Les composés de formule (I) tels que définis à l'une quelconque des revendications 1 à 3, dans lesquels

$R_1$ et $R_2$ forment ensemble avec l'atome d'azote auquel ils sont liés un radical hétérocyclique.

**5)** Les composés de formule (I) tels que définis à la revendication 4 dans lesquels le radical hétérocyclique est choisi dans le groupe constitué par les radicaux 1-pipéridinyle et hexahydro 1H-azépin-1-yle.

**6)** Les composés de formule (I) tels que définis à l'une quelconque des revendications 1 à 5 dans lesquels X représente un atome de soufre.

**7)** Les composés de formule (I) tels que définis à la revendication 1 dont les noms suivent :
4-hydroxy 1-[[4-(1-piperidinyl) phenyl] sulfonyl] 2-pyrrolidinethione
1-[[4-(hexahydro 1H-azepin-1-yl) phenyl] sulfonyl] 4-hydroxy 2-pyrrolidinone
1-[[4-(hexahydro 1H-azepin-1-yl) phenyl] sulfonyl] 4-hydroxy 2-pyrrolidinethione

**8)** A titre de médicaments, les composés de formule (I) tels que définis à l'une quelconque des revendications 1 à 6.

**9)** A titre de médicaments, les composés de formule (I) tels que définis à la revendication 7.

**10)** Les compositions pharmaceutiques renfermant comme principe actif au moins un médicament défini à l'une quelconque des revendications 8 et 9.

**11)** Procédé de préparation des composés de formule (I) tels que définis à l'une quelconque des revendications 1 à 6 caractérisé en ce que l'on soumet un composé de formule (II) :

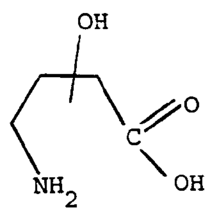

(II)

dans laquelle le radical OH est en position 2 ou 3 à l'action d'un composé de formule (III) :

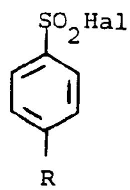

(III)

dans lequel Hal représente un atome d'halogène et R conserve sa signification précédente pour obtenir le composé de formule (IV) :

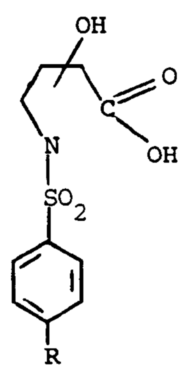

(IV)

dans laquelle OH est en position 2 ou 3 que l'on soumet à l'action d'un agent de cyclisation pour obtenir un composé de formule ($I_A$) :

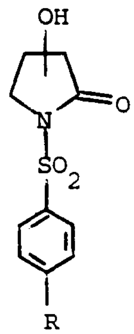

$(I_A)$

dans laquelle R a la signification précédente et OH est en position 3 ou 4, que l'on soumet, le cas échéant,

– soit à l'action d'un agent de fonctionnalisation du radical hydroxyle pour obtenir le composé de formule $(I_b)$ correspondant :

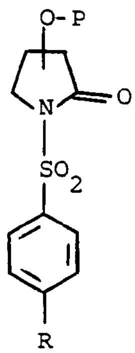

$(I_B)$

dans laquelle R a la signification précédente et P représente un groupement fonctionnel du radical hydroxyle,

– soit à l'action d'un agent de blocage de l'hydroxyle pour obtenir le composé de formule (V) :

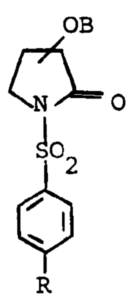

(V)

B représentant un groupement protecteur du radical hydroxyle, que l'on soumet à l'action d'un agent capable de convertir le radical carbonyle en radical thiocarbonyle pour obtenir le composé de formule (VI) :

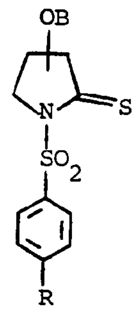

(VI)

que l'on soumet à l'action d'un agent de clivage du groupement protecteur du radical hydroxyle pour obtenir le composé de formule ($I_C$) correspondant :

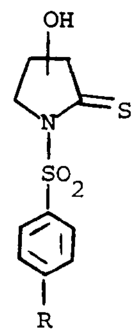

($I_C$)

dans laquelle R a la signification précédente et OH est en position 3 ou 4, que l'on soumet, le cas échéant, à l'action d'un agent de fonctionnalisation du groupement hydroxyle pour obtenir le composé de formule ($I_D$) correspondant.

**12)** Variante du procédé selon la revendication 11, caractérisée en ce que l'on soumet un composé de formule (VII) :

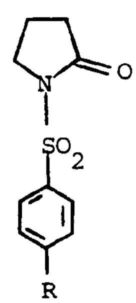

(VII)

dans laquelle R conserve sa signification précédente à l'action d'une base pour obtenir l'énolate de formule (VIII) :

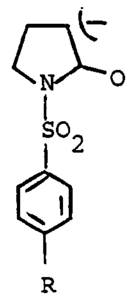

(VIII)

que l'on soumet à l'action d'un agent oxydant pour obtenir le composé de formule (I) correspondant dans lequel l'hydroxyle est libre, puis si désiré fonctionnalise, ou si désiré transforme le radical carbonyle en radical thiocarbonyle comme indiqué précédemment, puis si désiré fonctionnalise le composé obtenu.

**13)** A titre de produits chimiques nouveaux les produits de formule (IV), (V), (VI) et (VIII) obtenus lors de la mise en oeuvre du procédé des revendications 11 et 12.